# EUROPEAN PATENT SPECIFICATION

(11) **EP 1 721 893 B1**
(45) Date of publication and mention of the grant of the patent: **31.07.2013**
(21) Application number: 05720426.5
(22) Date of filing: 03.03.2005
(51) Int. Cl.: C07C 263/10, C07C 265/14, C07D 339/08

(54) **METHOD FOR PRODUCING ISOCYANATE COMPOUND**
VERFAHREN ZUR HERSTELLUNG EINER ISOCYANATVERBINDUNG
PROCEDE POUR PRODUIRE UN COMPOSE D'ISOCYANATE

(30) Priority: 05.03.2004 JP 2004062898
(43) Date of publication of application: 15.11.2006
(73) Proprietor: NIPPON SODA CO., LTD., Chiyoda-ku, Tokyo 100-8165 (JP)
(72) Inventor: WATAI, Toshiyuki, Takaoka-shi, Toyama 9338507 (JP)
(74) Representative: Wibbelmann, Jobst
(86) International application number: PCT/JP2005/004155
(87) International publication number: WO 2005/085186

(56) References cited:
- JP-A- 6 279 390
- JP-A- 7 309 827
- JP-A- 11 310 566
- JP-A- 2004 107 334
- US-A- 2 875 226
- "Experimental chemical course 20, ORGANIC SYNTHESIS II -alcohol, amine-", 6 July 1992 (1992-07-06), Chemical Society of Japan page 475,

## Description

### TECHNICAL FIELD

The present invention relates to a method for preparing an isocyanate compound and, more particularly, to a method for advantageously preparing an isocyanate compound industrially, by reacting a carbonate of a corresponding amine compound with phosgene.

### BACKGROUND ART

As a method for preparing an isocyanate compound by a phosgene method, a method of reacting a corresponding amine compound or a hydrochloride or carbonate thereof with phosgene in an inactive organic solvent has hitherto been known. For example, Japanese Unexamined Patent Application, First Publication No. Hei 7-309827 discloses a method of reacting an aliphatic polyamine or a hydrochloride or carbonate of the amine with phosgene in an inactive organic solvent while introducing an inactive gas into a reaction system. An object of this method is to efficiently prepare the objective isocyanate compound without producing tar, by reacting the aliphatic polyamine with phosgene while introducing an inactive gas into a reaction system.

However, even by this method, according to the kind of amine compound to be used or a salt thereof, the objective isocyanate compound could not be produced in a high yield. When an isocyanate compound as a raw material of lens is prepared, there is a case in which the reaction liquid is colored and coloration of the resulting isocyanate compound becomes a problem.

### DISCLOSURE OF THE INVENTION

Under these circumstances of these prior arts, the present invention has been completed and an object thereof is to provide a method for advantageously preparing a less colored isocyanate compound industrially.

The present inventors have intensively studied about a method for preparing an isocyanate compound by reacting a corresponding amine compound or a salt thereof with phosgene. As a result, they have found that, when a carbonate of an amine compound is used as a corresponding amine compound or a salt thereof, phosgene is introduced into an organic solvent solution of the carbonate, and the carbonate of the amine compound is reacted with phosgene, a less colored isocyanate compound can be prepared in a high yield by initiating blowing of phosgene at a low temperature no higher than the temperature at which the carbonate is decomposed, and heating to a predetermined temperature while blowing phosgene. Thus, the present invention has been completed.

According to the present invention, there is provided a method for preparing an isocyanate compound by introducing phosgene into an organic solvent solution of a carbonate of a corresponding amine compound and reacting the carbonate of the amine compound with phosgene, characterized in that phosgene is introduced into the organic solvent solution of the carbonate at a temperature of 0 to 80°C.

In the method according to the present invention, the isocyanate compound to be prepared is an aliphatic polyisocyanate compound.

In the method according to the present invention, phosgene is preferably introduced by blowing a gas.

In the method according to the present invention, the introduction of phosgene is initiated at 0 to 80°C, and the introduction of phosgene is more preferably initiated at 50°C or lower.

### BEST MODE FOR CARRYING OUT THE INVENTION

The method for preparing an isocyanate compound of the present invention is a method for preparing an isocyanate compound as defined in the claims by introducing phosgene into an organic solvent solution of a carbonate of a corresponding amine compound and reacting the carbonate of the amine compound with phosgene, characterized in that phosgene is introduced into the organic solvent solution of the carbonate at a temperature of 0 to 80°C and the isocyanate compound is an aliphatic polyisocyanate compound. .

The present invention can be carried out by the following procedure.
(i) First, the introduction of phosgene into an organic solvent solution prepared by dissolving or dispersing a carbonate of a corresponding amine in an inactive organic solvent (hereinafter referred to as a "reaction liquid", sometimes) is initiated.
(ii) Then, the introduction of phosgene into the reaction liquid is continued while gradually heating the reaction liquid to a fixed temperature.
(iii) Furthermore, phosgene is introduced while maintaining the temperature of the reaction liquid at a fixed temperature, and thus the reaction is completed.
(iv) After the completion of the reaction, degassing is conducted using nitrogen gas and the solvent is distilled off to obtain the objective product (isocyanate compound).

The amine compound used in the present invention is not particularly limited as long as it is a compound having an amino group in the molecule thereof, and may be a monoamine compound having one amino group or a polyamine compound having plural amino groups. In the present invention, a polyamine is preferable and an aliphatic polyamine is more preferable.

In the present invention, the corresponding amine compound means an amine compound corresponding to the objective isocyanate compound. For example, when the objective isocyanate compound is pentamethylene diisocyanate, the corresponding amine compound is pentamethylenediamine.

The amine compound used in the present invention is preferably a polyamine compound, and more preferably an aliphatic polyamine compound.

Examples of the aliphatic polyamine compound include cyclic aliphatic polyamines such as pentamethylenediamine, hexamethylenediamine, 2,2,4-trimethylhexamethylenediamine, 2,4,4-trimethylhexamethylenediamine, octamethylenediamine, nonamethylenediamine, and the like; aliphatic polyamines having an aromatic ring, such as o-xylylenediamine, m-xylylenediamine, p-xylylenediamine, mixtures of these isomers, and the like; cyclic aliphatic polyamines such as diaminocyclopentane, diaminocyclohexane, 1,3-bis(aminomethyl)cyclohexane, isophoronediamine, 1,4-bis(aminomethyl)norbornene, bis(4-aminocyclohexyl)methane, 2,2-bis(4-aminocyclohexyl)propane, 1,4-diaminocyclohexane, 1,3,5-triaminocyclohexane(1,3,5-cyclohexanetriamine), and the like; amino acid-based polyamines such as lysine methyl ester, lysine aminoethyl ester, and the like; and aliphatic polyamines having a heterocycle, such as 2,5-bis(aminomethyl)-1,4-dithiane, and the like.

The carbonate of the corresponding amine compound can be easily prepared by dissolving a corresponding amine compound in an inactive organic solvent and blowing carbon dioxide gas. Although the carbonate can be used after being isolated, the carbonate may be further reacted with phosgene without isolating the carbonate so as to prepare an isocyanate compound. According to the latter method, the objective isocyanate compound can be continuously prepared from the corresponding amine compound via the carbonate of the compound.

The inactive organic solvent used to prepare the carbonate of the amine compound is not particularly limited as long as it is inactive in the reaction. Usually, it is advantageous to use the same solvent as that used in the following reaction with phosgene in view of continuous industrial preparation.

The inactive organic solvent used in the reaction with phosgene is not particularly limited as long as it is inactive in the reaction. However, an inactive organic solvent having an extremely low boiling point is not preferable because the reaction proceeds slowly. Specific examples of the inactive organic solvent include aromatic hydrocarbons such as benzene, toluene, o-xylene, m-xylene, p-xylene, xylene mixtures thereof, and the like; aromatic halogenated hydrocarbons such as monochlorobenzene, o-dichlorobenzene, m-dichlorobenzene, p-dichlorobenzene, and the like; esters such as isoamyl acetate, n-butyl acetate, isobutyl acetate, and the like; and ethers such as dibutyl ether, ethylene glycol dimethyl ether, ethylene glycol diethyl ether, diethylene glycol dimethyl ether, and the like. These inactive organic solvents may be used alone or in combination of at least two kinds thereof.

The amount of organic solvent to be used varies depending on the kind of organic solvent or carbonate of the amine compound to be used and is usually from 1 to 10 liters, and preferably from 2.5 to 5 liters, with respect to 1 mol of the carbonate of the amine compound. When the amount of organic solvent is 1-Liter or less, it may become difficult to conduct stirring. However, the amount may be 1-Liter or less, provided that stirring can be conducted. Although the amount may be 10 liters or more, it is not preferred from an economical point of view.

The method of introducing phosgene into the reaction liquid is not particularly limited and examples thereof include a method of blowing the phosgene gas into the reaction liquid, a method of adding liquid phosgene to the reaction liquid, and a method of adding a multimer of phosgene to the reaction liquid to thereby generate phosgene in the reaction system. Among these methods, a method of blowing phosgene gas into the reaction liquid is preferable because the objective isocyanate compound can be efficiently obtained.

When the reaction is repeatedly conducted, as described hereinafter, a method of adding a solution containing recovered excess phosgene used last time to an organic solvent solution of a carbonate is advantageous.

The amount of phosgene used is usually 1 mol or more, preferably from 2 to 10 mols, and more preferably from 3 to 6 mols, based on 1 mol of an amino group of the carbonate of the amine compound as a raw material.

The temperature of the reaction liquid at the time of introducing phosgene (phosgene blowing initiation temperature) is 0 to 80°C, and more preferably 50°C or lower in view of the prevention of coloration of the isocyanate compound as the objective product. On the other hand, when the phosgene blowing initiation temperature is extremely low, the reaction does not proceed and therefore the phosgene blowing initiation temperature is controlled to a fixed temperature or higher. In the present invention, the phosgene blowing initiation temperature is preferably from 0 to 80°C, more preferably from 0 to 50°C, and particularly preferably from 20 to 40°C.

It is industrially preferred that, after a predetermined amount of phosgene is introduced at a temperature of 0 to 80°C so that the carbonate is not decomposed, preferably the temperature at which the introduction was initiated, heating-up is initiated. The amount of phosgene introduced before heating-up is not particularly limited, but is from 0.2 to 1.5 mols, and preferably about 1 mol, based on 1 mol of an amino group of the amine compound. When the amount is less than the above range, quality may deteriorate. On the other hand, when the amount is more than the above range, the amount of phosgene refluxed increases at the time of the following heating-up and the heating-up time increases, and therefore it is not preferred.

After initiating the introduction of phosgene or introducing a predetermined amount of phosgene, the temperature of the reaction liquid is gradually increased. The heating-up rate at this time is not particularly limited. However, since the concentration of phosgene in the reaction system is required to be maintained at a fixed concentration or more so as to suppress the decomposition of the carbonate of the amine compound, the heating-up rate of the reaction liquid is required to be appropriately adjusted by the amount of phosgene introduced per unit time.

Examples of the method of heating the reaction liquid include a method of heating the reaction liquid while changing the heating-up rate at a fixed rate or stepwise, and a method of maintaining the reaction liquid at a certain temperature for a fixed time in the middle of heating-up and then heating-up again, the former method being preferable. In the former method, after the temperature of the reaction liquid is increased to reach a fixed temperature (temperature (A)), blowing of phosgene is continued while maintaining the reaction liquid at the temperature. The temperature (A) varies depending on the kind of carbonate of the amine compound used, but is preferably higher than the phosgene blowing initiation temperature by at least 20°C. Although there is no upper limit of the temperature (A), the product is colored and impurities are produced when the temperature is extremely high. Therefore, it is not preferred.

The reaction can be allowed to smoothly proceed by forcibly discharging hydrogen chloride gas, which is generated in this heating-up process or at the time of blowing phosgene at the temperature (A), out of the system. The method of forcibly discharging the hydrogen chloride gas out of the system is not particularly limited and examples thereof include a method of slightly evacuating the system, a method of adjusting the amount of a coolant of a reflux condenser and discharging together with excess phosgene, and the like.

After the completion of the reaction, nitrogen gas or the like is blown at the same temperature and degassing is conducted. Then, insolubles are removed by filtration and the resulting filtrate is concentrated under reduced pressure to obtain the objective isocyanate compound. Purity of the objective product is 95% or more and a purification operation is not usually required. If a purification operation is further required, an isocyanate compound having higher purity can be obtained by conducting a distillation operation such as molecular distillation.

In the present invention, it is preferred to reuse phosgene, which is used in excess, from an economical or environmental point of view. Examples of the reusing method include a method of transferring an exhaust gas containing phosgene to the following reaction vessel and using again in the reaction, a method of utilizing the solvent distilled off after the completion of the reaction in the following reaction thereby utilizing phosgene dissolved in the solvent, and the like. An exhaust gas may be transferred to the solvent so that phosgene is absorbed in the solvent, and thereby phosgene can be reused. After the exhaust gas is absorbed in the solvent, phosgene can also be recovered from the solvent.

The isocyanate compound that can be prepared by the method of the present invention is an aliphatic polyisocyanate.

Specific examples of the aliphatic polyisocyanate that can be prepared by the method of the present invention include linear aliphatic polyisocyanates such as pentamethylene diisocyanate, hexamethylene diisocyanate, 2,2,4-trimethylhexamethylene diisocyanate, 2,4,4-trimethylhexamethylene diisocyanate, octamethylene diisocyanate, nonamethylene diisocyanate, and the like; aliphatic polyisocyanates having an aromatic ring, such as o-xylylene diisocyanate, m-xylylene diisocyanate, p-xylylene diisocyanate, mixtures of these isomers, and the like; cyclic aliphatic polyisocyanate such as cyclopentane diisocyanate, cyclohexane diisocyanate, 1,3-bis(isocyanate methyl)cyclohexane, isophorone diisocyanate, 1,4-bis(isocyanate methyl)norbornene, bis(4-isocyanate cyclohexyl)methane, 2,2-bis(4-isocyanate cyclohexyl)propane, 1,4-diisocyanate cyclohexane, 1,3,5-triisocyanate cyclohexane (1,3,5-cyclohexane triisocyanate), and the like; and heterocyclic aliphatic polyisocyanate, such as 2,5-bis(isocyanate methyl)-1,4-dithiane, and the like.

The method of the present invention is particularly useful as a method for preparing 2,5-bis(isocyanate methyl)-1,4-dithiane (hereinafter abbreviated to "BIMD"), or the like.

The present invention will now be described in more detail by way of examples and comparative examples, but the present invention is not limited to the following examples.

### Example 1: Preparation of 2,5-bis (isocyanate methyl)-1,4-dithiane

After 600 ml of dehydrated toluene and 27.36 g of BAMD (2,5-bis(aminomethyl)-1,4-dithiane) were put into a 1-L flask equipped with a reflux condenser, a thermometer, a gas blowing tube and a stirrer, 20.2 g of carbon dioxide gas was blown at 25 to 30°C over one hour to obtain a carbonate. After the completion of blowing of the carbon dioxide gas, blowing of phosgene gas was initiated at 25 to 30°C, and the temperature of the reaction liquid was increased to a temperature within a range from 90°C to 100°C over 4 hours while blowing phosgene gas at 15 g/hour for 4 hours. After the temperature of the reaction liquid reached a temperature within a range from 90°C to 100°C, phosgene gas was further blown at 15 g/hour for 6 hours. After the completion of the reaction, degassing was conducted by blowing nitrogen for one hour, followed by filtration and further concentration of the filtrate to obtain 35.3 g of a crude BIMD. Purity of BIMD was 96.8% and the yield in terms of a pure product was 99%. The resulting BIMD was scarcely colored.

### Example 2: Preparation of 2,5-bis (isocyanate methyl)-1,4-dithiane

After 600 ml of dehydrated toluene and 36.39 g of BAMD (2,5-bis(aminomethyl)-1,4-dithiane) were put into a 1-L flask equipped with a reflux condenser, a thermometer, a gas blowing tube and a stirrer, 26.4 g of carbon dioxide gas was blown at 25 to 30°C over one hour to obtain a carbonate. After the completion of blowing of the carbon dioxide gas, blowing of phosgene gas was initiated at 25 to 30°C, and the temperature of the reaction liquid was increased to a temperature within a range from 90°C to 100°C over 4 hours while blowing phosgene gas at 20 g/hour for 4 hours. After the temperature of the reaction liquid reached a temperature within a range from 90°C to 100°C, phosgene gas was blown at 20 g/hour for 3 hours and was further blown at 10 g/hour for 3 hours. After the completion of the reaction, degassing was conducted by blowing nitrogen for one hour, followed by filtration and further concentration of the filtrate to obtain 48.3 g of a crude BIMD. Purity of BIMD was 95.3% and the yield in terms of a pure product was 98.8%. The resulting BIMD was scarcely colored.

### Example 3: Preparation of 2,5-bis(isocyanate methyl)-1,4-dithiane

After 600 ml of dehydrated toluene and 27.379 g of BAMD (2,5-bis(aminomethyl)-1,4-dithiane) were put into a 1-L flask equipped with a reflux condenser, a thermometer, a gas blowing tube and a stirrer, 19.9 g of carbon dioxide gas was blown at 25 to 30°C over one hour to obtain a carbonate. After the completion of blowing of the carbon dioxide gas, 59.4 g of phosgene gas was blown at the same temperature over 2 hours. Subsequently, the temperature was increased from the same temperature to a temperature within a range from 90°C to 100°C over 4 hours while blowing phosgene gas at 7.4 g/hour for 4 hours. After the temperature reached a temperature within a range from 90°C to 100°C, phosgene gas was further blown at 7.4 g/hour for 8 hours. After the completion of the reaction, degassing was conducted by blowing nitrogen for one hour, followed by filtration and further concentration of the filtrate to obtain 35.95 g of a crude BIMD. Purity of BIMD was 94.3% and the yield in terms of a pure product was 98.1%. The resulting BIMD was scarcely colored.

### Comparative Example 1: Preparation of 2,5-bis(isocyanate methyl)-1,4-dithiane

After 600 ml of dehydrated toluene and 27.38 g of BAMD (2,5-bis(aminomethyl)-1,4-dithiane) were put into a 1-L flask equipped with a reflux condenser, a thermometer, a gas blowing tube and a stirrer, 20.2 g of carbon dioxide gas was blown at 25 to 30°C over one hour to obtain a carbonate. After the completion of blowing of the carbon dioxide gas, the temperature was increased from the same temperature to a temperature of 90 to 100°C. During heating-up, phosgene was not blown. After heating-up, phosgene gas was blown at 15 g/hour for 6 hours while maintaining the same temperature. As a result, a substance having high viscosity began to deposit on a stirring blade and the wall surface of a reaction vessel and therefore the reaction was terminated. Degassing was conducted by blowing nitrogen for one hour, followed by filtration and further concentration of the filtrate to obtain 5.06 g of a crude BIMD. Purity of BIMD was 39.1% and the yield in terms of a pure product was 5.7%. The resulting BIMD was colored yellow.

### Comparative Example 2: Preparation of 2,5-bis(isocyanate methyl)-1,4-dithiane

After 625 ml of monochloro benzene and 45.6 g of BAMD (2,5-bis(aminomethyl)-1,4-dithiane) were put into a 1-L flask equipped with a reflux condenser, a thermometer, a gas blowing tube and a stirrer, 23.7 g of a hydrochloric acid gas was blown at 90°C over one hour to obtain a hydrochloride. After the completion of blowing of the hydrochloric acid gas, the temperature was increased to a temperature within a range from 125 to 130°C. After heating-up, phosgene gas was blown at 17.3 g/hour for 16 hours while maintaining the same temperature. Furthermore, phosgene gas was blown at 4.9 g/hour for 2 hours and the reaction was completed. Degassing was conducted by blowing nitrogen for one hour, followed by filtration and further concentration of the filtrate to obtain 58.9 g of a crude BIMD. Purity of BIMD was 94.6% and the yield in terms of a pure product was 96.5%. The resulting BIMD was colored yellow.

The results of a comparison between Example 1 (in the case of using a carbonate) and Comparative Example 2 (in the case of using a hydrochloride) are shown in Table 1.

**Table 1**

| | Reaction time (hours) | Total amount of phosgene (mol folds / theory) | Purity (%) | Yield (%) |
|---|---|---|---|---|
| Example 1 | 10 | 10.0 | 96.8 | 99.0 |
| Comparative Example 2 | 18 | 11.3 | 94.6 | 96.5 |

As is apparent from the results shown in Table 1, the reaction time of Example 1 was shorter than that in Comparative Example 2 and also the amount of phosgene used in Example 1 was smaller than that in Comparative Example 2. The isocyanate compound (BIMD) obtained in Example 1 showed high purity and good yield as compared with the BIMD obtained in Comparative Example 2.

### Example 4: Preparation of hexamethylene diisocyanate

After 660 ml of dehydrated toluene and 25.9 g of hexamethylenediamine were put into a 1-L flask equipped with a reflux condenser, a thermometer, a gas blowing tube and a stirrer, 32.6 g of carbon dioxide gas was blown at 25 to 30°C over 2 hours to obtain a carbonate. After the completion of blowing of the carbon dioxide gas, blowing of phosgene gas was initiated at 25 to 30°C, and the reaction temperature was increased to 90°C over 5 hours while blowing phosgene gas at 12 g/hour. After the reaction temperature reached 90°C, phosgene gas was blown at 10 to 12 g/hour for 8 hours, and then phosgene gas was further blown at 14 g/hour for 4 hours. After the completion of the reaction, degassing was conducted by blowing nitrogen gas for one hour, followed by filtration and further concentration of the filtrate to obtain 34.09 g of hexamethylene diisocyanate. Purity of hexamethylene diisocyanate was 86.4% and the yield in terms of a pure product was 87.5%. The resulting hexamethylene diisocyanate was scarcely colored.

### Example 5: Preparation of bis(isocyanate methyl)cyclohexane

After 720 ml of dehydrated toluene and 17.2 g of 1,3-bis(aminomethyl)cyclohexane were put into a 1-L flask equipped with a reflux condenser, a thermometer, a gas blowing tube and a stirrer, 16 g of carbon dioxide gas was blown at 25 to 30°C over one hour and 20 minutes to obtain a carbonate. After the completion of blowing of the carbon dioxide gas, blowing of phosgene gas was initiated at 25 to 30°C, and the reaction temperature was increased to 90°C over 3.5 hours while blowing phosgene gas at 12 g/hour. After the temperature reached 90°C, phosgene gas was blown at 12 to 15 g/hour for 19.5 hours. After the completion of the reaction, degassing was conducted by blowing nitrogen gas for 1.5 hours, followed by filtration and further concentration of the filtrate to obtain 22.4 g of 1,3-bis(isocyanate methyl)cyclohexane. Purity of 1,3-bis(isocyanate methyl)cyclohexane was 82.5% and the yield in terms of a pure product was 79.2%. The resulting 1,3-bis(isocyanate methyl)cyclohexane was scarcely colored.

### Example 6: Preparation of m-xylylene diisocyanate

After 720 ml of dehydrated toluene and 16.35 g of m-xylylenediamine were put into a 1-L flask equipped with a reflux condenser, a thermometer, a gas blowing tube and a stirrer, 80 g of carbon dioxide gas was blown at 30 to 35°C over one hour to obtain a carbonate. After the completion of blowing of the carbon dioxide gas, phosgene gas was blown at 30 to 35°C at 48 g/hour for 0.5 hours. Then, the temperature of the reaction liquid was increased to 95°C over 5 hours and also phosgene gas was blown at 12 g/hour for 4 hours. After the temperature reached 95°C, phosgene gas was further blown at 12 g/hour for 2.5 hours. After the completion of the reaction, degassing was conducted by blowing nitrogen gas for 1.5 hours, followed by filtration and further concentration of the filtrate to obtain 23.70 g of crude m-xylylene diisocyanate. Purity of m-xylylene diisocyanate was 91.4% and the yield in terms of a pure product was 96.0%. The resulting m-xylylene diisocyanate was scarcely colored.

### Example 7: Preparation of 2,5-bis(isocyanate methyl)-1,4-dithiane

After 4600 L of toluene and 360 Kg of BAMD (2,5-bis(aminomethyl)-1,4-dithiane) were put in a 6-m³ dissolution vessel at 20°C and then dissolved, the mixture was transferred to a 10-m³ reaction vessel. 132 Kg of carbon dioxide gas was blown over 2 hours to obtain a carbonate. After the completion of blowing of the carbon dioxide gas, 2500 L of a toluene solution containing about 200 Kg of the recovered phosgene was added at 25 to 30°C, blowing of phosgene gas was initiated at the same temperature, and also heating-up was initiated at the same time. Heating-up was conducted from 30 to 90°C over 3 hours. Then, the reaction was conducted at 90 to 110°C for 17 hours. During the reaction, phosgene was blown at a rate of 100 Kg/hour for 6 hours after the initiation of heating-up, and was blown at a rate of 130 kg/hour after 6 hours had passed since the initiation of heating-up. The system was slightly evacuated to -1 to -4.5 kPa. The amount of coolant of a reflux condenser was controlled and hydrogen chloride gas and a portion of phosgene were discharged out of the system. After the completion of the reaction, degassing was conducted by blowing nitrogen gas at the same temperature for 3 hours, the reaction solution was cooled and toluene was distilled off under reduced pressure to obtain 467.6 Kg of BIMD4. Purity was 96.0% and the yield in terms of a pure product was 97.5%.

### INDUSTRIAL APPLICABILITY

Since the method of the present invention enables the reaction to proceed at low temperature and also shortens the reaction time, a less colored isocyanate compound can be efficiently prepared. As compared with a method using a hydrochloride of an amine compound, not only the objective product can be obtained in a high yield, but also the amount of phosgene used can be reduced. Therefore, it can be said that the method of the present invention is an industrially advantageous method for preparing an isocyanate compound.

## Claims

1. A method for preparing an isocyanate compound by introducing phosgene into an organic solvent solution of a carbonate of a corresponding amine compound and reacting the carbonate of the amine compound with phosgene, **characterized in that** phosgene is introduced into the organic solvent solution of the carbonate at a temperature of 0 to 80°C and the isocyanate compound is an aliphatic polyisocyanate compound.

2. The method according to claim 1, wherein the introduction of phosgene is initiated at 50 °C or lower.

3. The method according to claim 1 or 2, wherein the amount of phosgene introduced before heating-up is initiated is within a range from 0.2 to 1.5 mols based on 1 mol of an amino group of the amine compound.

4. The method according to claim 1, wherein hydrogen chloride produced as byproduct by the reaction is forcibly discharged out of the system.

5. The method according to claim 1, wherein heating-up is initiated after a predetermined amount of phosgene is introduced while maintaining the temperature of 0 to 80°C.

6. The method according to claim 1, wherein heating-up is initiated after a predetermined amount of phosgene is introduced while maintaining the temperature of 0 to 50°C.

## Patentansprüche

1. Verfahren zur Herstellung einer Isocyanat-Verbindung durch Einführen von Phosgen in eine organische Lösungsmittellösung eines Carbonats einer entsprechenden Amin-Verbindung und Umsetzen des Carbonats der Amin-Verbindung mit Phosgen, **dadurch gekennzeichnet, dass** Phosgen in die organische Lösungsmittellösung des Carbonats bei einer Temperatur von 0 bis 80 °C eingeführt wird und die Isocyanat-Verbindung eine aliphatische Polyisocyanat-Verbindung ist.

2. Verfahren nach Anspruch 1, bei dem mit der Einführung von Phosgen bei 50 °C oder weniger begonnen wird.

3. Verfahren nach Anspruch 1 oder 2, bei dem die Menge an Phosgen, die eingeführt wird, bevor mit dem Erwärmen begonnen wird, in einem Bereich von 0,2 bis 1,5 Mol, bezogen auf 1 Mol einer Aminogruppe der Amin-Verbindung, liegt.

4. Verfahren nach Anspruch 1, bei dem Chlorwasserstoff, der als Nebenprodukt durch die Reaktion erzeugt wird, unter Zwang aus dem System ausgetragen wird.

5. Verfahren nach Anspruch 1, bei dem mit dem Erwärmen begonnen wird, nachdem eine vorbestimmte Menge an Phosgen eingeführt worden ist, während eine Temperatur von 0 bis 80 °C beibehalten wurde.

6. Verfahren nach Anspruch 1, bei dem mit dem Erwärmen begonnen wird, nachdem eine vorbestimmte Menge an Phosgen eingeführt worden ist, während eine Temperatur von 0 bis 50 °C beibehalten wurde.

## Revendications

1. Procédé de préparation d'un composé isocyanate par introduction de phosgène dans une solution dans un solvant organique d'un carbonate d'un composé amine correspondant et réaction du carbonate du composé amine avec le phosgène, **caractérisé en ce que** le phosgène est introduit dans la solution dans un solvant organique du carbonate à une température de 0 à 80 °C et le composé isocyanate est un composé polyisocyanate aliphatique.

2. Procédé selon la revendication 1, dans lequel l'introduction du phosgène est initiée à 50 °C ou moins.

3. Procédé selon la revendication 1 ou 2, dans lequel la quantité de phosgène introduite avant que le chauffage ne soit initié est dans une plage de 0,2 à 1,5 mole sur la base de 1 mole d'un groupe amino du composé amine.

4. Procédé selon la revendication 1, dans lequel le chlorure d'hydrogène produit comme sous-produit par la réaction est évacué de force hors du système.

5. Procédé selon la revendication 1, dans lequel le chauffage est initié après qu'une quantité prédéterminée de phosgène a été introduite tout en maintenant la température de 0 à 80 °C.

6. Procédé selon la revendication 1, dans lequel le chauffage est initié après qu'une quantité prédéterminée de phosgène a été introduite tout en maintenant la température de 0 à 50 °C.
